(19) Europäisches Patentamt
European Patent Office
Office européen des brevets

(11) EP 0 623 347 B1

(12) # FASCICULE DE BREVET EUROPEEN

(45) Date de publication et mention de la délivrance du brevet:
**31.10.2001 Bulletin 2001/44**

(51) Int Cl.[7]: **A61K 31/663**, A61K 31/675, A61P 19/00

(21) Numéro de dépôt: **94400719.4**

(22) Date de dépôt: **01.04.1994**

(54) **Utilisation de dérivés d'acide bisphosphonique pour la préparation de médicaments destinés à favoriser la réparation osseuse**

Verwendung von Bisphosphorsäurederivaten zur Herstellung eines Medikamentes zur Förderung der Knochenheilung

Use of bisphosphoric acid derivatives for the manufacture of a medicament to improvement bone repair

(84) Etats contractants désignés:
**AT BE CH DE DK ES FR GB GR IE IT LI LU NL PT SE**

(30) Priorité: **05.04.1993 FR 9303999**

(43) Date de publication de la demande:
**09.11.1994 Bulletin 1994/45**

(73) Titulaire: **SANOFI-SYNTHELABO**
**75013 Paris (FR)**

(72) Inventeurs:
- **Barbier, Alain**
  **F-34980 Saint Clement la Riviere (FR)**
- **Lacheretz, Frédéric**
  **F-34570 Pignan (FR)**

(74) Mandataire: **Le Roux, Martine et al**
**Cabinet Beau de Loménie**
**158, rue de l'Université**
**75340 Paris Cédex 07 (FR)**

(56) Documents cités:

| | |
|---|---|
| **EP-A- 0 186 405** | **EP-A- 0 325 482** |
| **EP-A- 0 336 851** | **EP-A- 0 600 834** |
| **EP-A- 0 689 443** | **WO-A-86/00902** |

- **J BONE MINER RES (UNITED STATES), JUN 1992, VOL. 7, NO. 6, PAGE(S) 599-609, Geusens P et al 'Longitudinal effect of tiludronate on bone mineral density, resonant frequency, and strength in monkeys.'**
- **CLIN PHARM (UNITED STATES), JUL 1989, VOL. 8, NO. 7, PAGE(S) 485-95, Stumpf JL 'Pharmacologic management of Paget's disease.'**
- **J CLIN ENDOCRINOL METAB (UNITED STATES), NOV 1985, VOL. 61, NO. 5, PAGE(S) 952-6, Hoekman K et al 'Characteristics and bisphosphonate treatment of a patient with juvenile osteoporosis.'**
- **ARCH ORTHOP TRAUMA SURG, vol.112, no.5, 21 Septembre 1993 pages 228 - 231 M.T. NYMAN 'Clodronate increases the calcium content in fracture callus'**
- **OSTEOPOROS INT, vol.3, no.3, 1993 pages S29 - S39 D.M. BLACK 'Design of the fracture intervention trial'**
- **J ORTHOP RES, vol.3, no.4, 1985 pages 499 - 507 T.M. LENEHAN ET AL. 'Effect of EHDP on fracture healing in dogs'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet l'utilisation de dérivés de l'acide bisphosphonique pour la préparation de médicaments destinés à favoriser la réparation osseuse en médecine humaine ou vétérinaire.

Le processus physiologique de réparation osseuse se définit par l'apparition successive de tissus cicatriciels différents qui sont, par ordre d'apparition : le cartilage, l'os primaire (non organisé) puis l'os lamellaire (organisé). Chacun d'entre eux ne se forme qu'après la destruction du précédent. Un tel remaniement est donc consécutif à une résorption qui est assurée par des cellules macrophagiques : les chondroclastes pour la résorption du cartilage et les ostéoclastes pour la résorption de l'os. Ceci est bien décrit dans : Le tissu osseux, édité sous la direction de L. Teot, J. Vidal., J. Dossa, collection : Biologie de l'appareil locomoteur, Diffusion Vigot, 1989 et par H.M. Frost dans The biology of fracture healing. An overview for clinicians, I and II, Clin. Orthop., 1989, 248, 283..

Dans la description et dans les revendications qui vont suivre, par dérivé d'acide bisphosphonique, on entend un composé de formule :

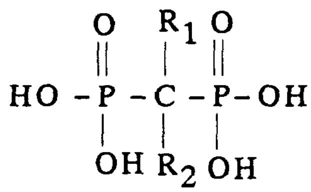

(I)

dans laquelle :

- $R_1$ représente un atome d'hydrogène, un atome d'halogène, un hydroxyle, un amino, un monoalkyl($C_1$-$C_4$) amino, un dialkyl($C_1$-$C_4$)amino ;
- $R_2$ représente un atome d'halogène, un alkyle linéaire comportant de 1 à 5 atomes de carbone non substitué ou substitué par un groupe choisi parmi un atome de chlore, un hydroxyle, un amino, un monoalkyl ($C_1$-$C_4$) amino, un dialkyl($C_1$-$C_4$)amino, un cycloalkyl($C_3$-$C_7$) amino,

ou $R_2$ représente un phénoxy, un phényle, un thiol, un phénylthio, un chlorophénylthio, un pyridyle, un pyridylméthyle, un 1-pyridyl-1-hydroxyméthyle, un imidazolyl-méthyle, un thiomorpholin-4-yle ;
et ses sels avec des acides minéraux ou organiques pharmaceutiquement acceptables.

Ces composés sont connus, ils ont été décrits comme médicaments dans le traitement des affections osseuses, notamment dans les brevets ci-après : BE 902308, BE 865434, DE 2130794, US 4134969, EP 162510, FR 2525223, EP 39033, US 4578376, EP 203549, BE 822930, US 4621077, JP 55-98193, EP 186405, EP 100718, WO 86/00902, WO 87/03598, US 4922007, EP 304961, JP 63-150291, EP 325482.

Parmi ces dérivés d'acide bisphosphonique, on peut citer en particulier les composés suivants:

- l'acide 1-hydroxy éthylidène bisphosphonique dont la dénomination commune internationale est l'acide étidronique, et ses sels de sodium ;
- l'acide 2-pyridin-2-yl éthylidène bisphosphonique dont la dénomination commune internationale est l'acide piridronique et ses sels de sodium ;
- l'acide dichlorométhylène bisphosphonique dont la dénomination commune internationale est l'acide clodronique et ses sels de sodium ;
- l'acide 3-amino-1-hydroxy propylidène bisphosphonique dont la dénomination commune internationale est l'acide pamidronique et ses sels de sodium ;
- l'acide 4-amino-1-hydroxy butylidène bisphosphonique dont la dénomination commune internationale est acide alendronique et ses sels de sodium ;
- l'acide 6-amino-1-hydroxy hexylidène bisphosphonique et ses sels ;
- l'acide phénoxy méthylène bisphosphonique et ses sels ;
- l'acide thiomorpholino méthylène bisphosphonique et ses sels ;
- l'acide 4-chloro phénylthiométhylène bisphosphonique dont la dénomination commune est acide tiludronique et ses sels pharmaceutiquement acceptables, notamment le sel disodique ;
- l'acide 1-hydroxy-2-(pyridin-3-yl)éthylidène bisphosphonique dont la dénomination commune internationale est acide risédronique et ses sels de sodium ;
- l'acide 1-hydroxy-2-(imidazol-2-yl)éthyl-1,1-bisphosphonique et ses sels ;
- l'acide (cycloheptylamino)méthylène bisphosphonique et ses sels ;

- l'acide 2-hydroxyéthylidène-2-(pyridin-3-yl)-1,1-bisphosphonique et ses sels de sodium.

D'une manière générale, on appelle bisphosphonates, les sels des dérivés d'acide bisphosphonique mentionnés ci-dessus.
Selon la présente invention, l'utilisation de l'acide tiludronique et de ses sels pharmaceutiquement acceptables, notamment le sel disodique est particulièrement préférée.
L'effet biologique des dérivés d'acide bisphosphonique est d'inhiber la résorption osseuse en diminuant l'activité des ostéoclastes, comme cela apparait dans les publications ci-après :

- H. Fleisch, R.G.G. Russel, M.D. Francis : Diphosphonates inhibit hydroxyapatite dissolution in vitro and bone resorption in tissue culture and in vivo ; Science, 1969, 165, 1262-1264 ;
- P.M. Boonekamp, L.J.A. Van der Wee-Pals, M.M.L. Van Wijk-Lennep, C.W. Thesing, O.L.M. Bijvoet : Two modes of action of bisphosphonates on osteoclastic resorption of mineralized matrix ; Bone Miner., 1986, 1, 27-39 ;
- A.M. Flanaghan, T.J. Chambers : Dichloromethylene bisphosphonate ($Cl_2$, MBP) inhibits bone resorption through injury to osteoclasts that resorb ClMBP coated bone. Bone Miner., 1989, 6, 33.

Plusieurs dérivés d'acide bisphosphonique sont actuellement en développement chez l'homme ou commercialisés pour être utilisés dans le traitement de maladies osseuses comme la maladie de Paget et l'ostéoporose. Ces maladies sont caractérisées par une stimulation ostéoclastique (plus importante encore dans la maladie de Paget que dans l'ostéoporose) qu'il est nécessaire de freiner.
Un article de H.C. Tennenbaum et al. publié dans Bone, 1992, 13, 249-255, fait état d'une augmentation in vitro des marqueurs enzymatiques de l'ostéogénèse après administration de bisphosphonates à faible dose.
Un article de Feretti et al. publié dans Bone Miner., 1990, 11, 111-122, relate une amélioration des propriétés biomécaniques de l'os, sans augmentation de la masse osseuse, observée ex-vivo après administration de bisphosphonates.
Par ailleurs, comme les bisphosphonates inhibent la résorption osseuse et comme l'étape de résorption est indispensable dans le processus de réparation osseuse qui se produit à la suite d'une fracture ou d'une intervention en chirurgie osseuse, on ne pouvait pas espérer un effet bénéfique de l'administration des bisphosphonates dans la réparation osseuse et on pouvait même s'attendre à ce que les bisphosphonates soient contre-indiqués dans le cas de réparation osseuse. Ainsi on peut citer F. Bonnel et B. Tachot : Biologie de la cicatrisation osseuse des fractures *in* Le Tissu Osseux (op. cité : édité sous la direction de L. Teot, J. Vidal, J. Dossa, collection: Biologie de l'appareil locomoteur, Diffusion Vigot, 1989) : "Les Diphosphonates. Ils ont pour effet de freiner la résorption ostéoclastique et dépriment le remodelage osseux dans son ensemble. Ils doivent être arrêtés en cas de fracture jusqu'à consolidation".
Pourtant, il a été trouvé de façon tout-à-fait inattendue, que les dérivés de l'acide bisphosphonique sont utiles dans la réparation osseuse, notamment pour l'accélération de celle-ci.
Ainsi la présente invention a pour objet l'utilisation de dérivés de l'acide bisphosphonique pour la préparation de médicaments destinés à favoriser la réparation osseuse, notamment suite à une fracture ou à une intervention de chirurgie osseuse.
De tels médicaments peuvent être utilisés en médecine humaine et en médecine vétérinaire.
De tels médicaments peuvent être administrés par différentes voies d'administration, par exemple par voie orale, par voie parentérale, par administration percutanée, ou au moyen d'un implant.
Lorsque l'on prépare un médicament pour l'administration par voie orale, on peut utiliser tout excipient convenable et en particulier un excipient qui facilite l'absorption du médicament comme le laurylsulfate de sodium.
Les doses d'administration du médicament selon l'invention dépendent du dérivé d'acide bisphosphonique utilisé, du mode d'administration et de l'importance de l'effet recherché sur la réparation osseuse.
Le médicament selon l'invention peut être administré en dose unique ou répétée. Pour l'administration en dose répétée, on peut choisir une administration continue quotidienne, 1 à 3 fois par jour, pendant le temps que dure la réparation de fracture (un à plusieurs mois) ou une administration intermittente, par exemple 1 jour par semaine pendant un à plusieurs mois.
L'unité de dosage peut comprendre de 0,001 mg à 400 mg de dérivé(s) d'acide bisphosphonique de formule (I), plus particulièrement 0,01 mg à 400 mg.
Ainsi les doses d'administration du médicament préparé selon l'invention peuvent varier de 0,001 mg à 1,2 g par jour, plus particulièrement de 0,01 mg à 1,2 g par jour.
De préférence l'unité de dosage comprend de 0,1 à 250 mg de dérivé(s) d'acide bisphosphonique de formule (I).
Pour une administration par voie orale, la composition pharmaceutique selon l'invention peut être sous forme de comprimé, gélule, poudre, granulé, gouttes ou toute autre forme administrable par voie orale.
La composition selon l'invention peut contenir en outre des ingrédients utilisés habituellement en pharmacie

pour la préparation de formes orales. Ainsi la composition selon l'invention peut contenir un agent de désintégration, un agent d'écoulement, un lubrifiant et tout excipient de masse convenable.

Comme excipient de masse, on peut utiliser le lactose, la cellulose ou les amidons. Comme lubrifiant on peut utiliser l'acide stéarique, le stéarate de magnésium, la L-leucine ou, par exemple, le tribéhénate de glycérol. Comme agent de désintégration, on peut utiliser le carboxyméthylamidon sodique, la carboxyméthylcellulose sodique réticulée ou, par exemple, la polyvinylpyrrolidone réticulée. Comme agent d'écoulement, on peut utiliser la silice pure ou le dioxyde de silicium colloïdal.

La présente invention se réfère également aux formes orales à dissolution instantanée et aux formes orales effervescentes obtenues en ajoutant un couple effervescent à la composition selon l'invention. Comme couple effervescent, on peut utiliser, par exemple, l'acide tartrique et le bicarbonate de sodium ou l'acide citrique et le bicarbonate de sodium.

La forme comprimé est une forme préférée selon l'invention. L'invention se réfère également aux comprimés à dissolution instantanée, aux comprimés effervescents ainsi qu'aux comprimés recouverts d'un enrobage. Une composition contenant du laurylsulfate de sodium selon le brevet européen EP 336851 est particulièrement convenable.

Pour une administration par voie orale du tiludronate la dose unitaire varie de 0,05 mg à 1000 mg, avantageusement de 0,05 mg à 400 mg, plus particulièrement de 0,1 mg à 250 mg.

Pour une administration par voie orale du pamidronate ou de l'étidronate la dose unitaire varie de 0,05 mg à 1 g, avantageusement de 0,05 mg à 400 mg.

Pour une administration par voie orale de l'alendronate, du risedronate, ou de l'acide (cycloheptylamino) méthylène bisphosphonique ou un de ses sels, la dose journalière, administrée varie de 0,001 mg à 100 mg, de façon préférentielle de 0,01 mg à 100 mg.

Pour une administration parentérale, on utilise des suspensions aqueuses, des solutions salines isotoniques ou des solutions stériles et injectables qui peuvent contenir des agents de dispersion et/ou des agents mouillants pharmacologiques compatibles, par exemple le propylèneglycol ou le butylèneglycol.

Pour une administration par voie percutanée, la composition selon l'invention peut être sous forme d'une crème, d'une pommade ou d'une composition pour administration transdermique.

EXEMPLE 1 : Mesure de la réparation osseuse.

Une étude expérimentale de réparation osseuse a été réalisée chez le chien en utilisant un modèle d'hémiostéotomie.

Le principe de l'hémiostéotomie est de produire un trait de fracture incomplet au niveau de la diaphyse d'un os long (ulna) appartenant à un double rayon osseux. La demi-fracture est ainsi stabilisée par la partie non fracturée du même os et par le deuxième os, évitant toute utilisation de matériel de fixation.

Trois groupes composés de quatre chiens Beagle mâles ont été utilisés et respectivement traités une fois par jour par gavage pendant six semaines (J 0 à J 45) à partir de l'hémiostéotomie. Les produits sont administrés sous forme de gélules.

TABLEAU 1

| Groupe | Nombre d'animaux | Produit | Dose (mg/kg) |
|---|---|---|---|
| 1 | 4 | véhicule (gélules vides) | - |
| 2 | 4 | tiludronate | 5 |
| 3 | 4 | étidronate | 10 |

Une double administration de tétracycline a été réalisée à plusieurs jours d'intervalle pour marquer le trait de minéralisation à deux temps différents aux jours J 26 et J 27 puis J 41 et J 42.

Au jour J 45 après l'hémiostéotomie, les animaux ont été sacrifiés. Les os hémiostéotomisés ont été prélevés et fixés pour l'examen histomorphométrique suivant :

- mesure du nombre d'ostéoclastes par $mm^2$,
- mesure de surface active de résorption (%) : il s'agit de la surface occupée par des ostéoclastes par rapport à la surface totale. Cette mesure permet d'évaluer la résorption osseuse,
- mesure de la surface du double marquage (%) par rapport à la surface totale de l'os: cette mesure permet de quantifier l'os lamellaire,
- mesure du volume trabeculaire osseux (TV), exprimé en pourcentage du volume osseux total (BV). Ceci représente, à la rupture créée par l'hemiostéotomie, la quantité d'os formée, incluant le tissu osteoïde et le tissu minéralisé.

Au jour J45 après l'hemiostéotomie, on effectue également des tests biomécaniques par l'analyse de la fréquence de résonnance : les fréquences de résonnance sont mesurées sur l'ulna dans le plan longitudinal, les vibrations sont enregistrées par un microphone placé 1 cm au dessus de l'os, la stimulation est produite par l'impact d'un marteau à la moitié de la longueur de l'ulna. Les signaux sont transmis à un analyseur de spectre qui donne la fréquence du spectre et on détermine les fréquences de résonnance.
La moyenne de 4 mesures est calculée pour les signaux enregistrés. Deux paramètres sont ainsi évalués :

1) Rigidité osseuse : évaluation de la résistance osseuse à des vibrations dans le plan longitudinal.
   La rigidité calculée est : $F^2M$ en $Hz^2xg$.
   F : fréquence de résonnance (Hertz).
   M : masse (gramme).
2) Résistance à la rupture : calcul de la force opposée aux vibrations dans le plan longitudinal.

La résistance à la rupture est $F^2ML$ en $Hz^2xg$ xmm.

- L : longueur (mm).

Les résultats (moyenne et erreur standard ramenée à la moyenne : e.s.m.) sont présentés dans les tableaux ci-après :

TABLEAU 2

| Groupe (produit) | Nombre d'ostéoclastes par $mm^2$ (e.s.m.) | Surface active de résorption (%) (e.s.m.) | Surface de double marquage (%) (e.s.m.) |
|---|---|---|---|
| Groupe 1 (témoin) | 8,19 (1,19) | 3,32 (0,35) | 2,21 (1,48) |
| Groupe 2 (tiludronate) | 9,54 (1,73) | 5,44 (0,61) | 20,55* (2,96) |
| Groupe 3 (étidronate) | 5,58 (0,92) | 4,95 (0,18) | 6,49 (4,12) |

* : $p < 0,05$.

Les résultats montrent clairement une augmentation de la quantité d'os lamellaire définitif pour les deux bisphosphonates alors que, parallèlement, les paramètres de résorption osseuse n'ont pas été modifiés.

TABLEAU 3

| Groupe (produit) | Volume trabeculaire osseux BV/TV % (e.s.m.) | Rigidité osseuse $Hz^2xg$ (e.s.m.) | Résistance à la ruptur $Hz^2xg$ xmm(e.s.m.) |
|---|---|---|---|
| Groupe 1 (témoin) | 65,5 (2,6) | 85,9 (16,9) | 101,8 (17,1) |
| Groupe 2 (tiludronate) | 70,4 (0,9) | 157,4 (26,7) | 192,8 (32,5) |
| Groupe 3 (étidronate) | 60,3 (6,2) | 110,8 (25,9) | 135,4 (34,1) |

Ces résultats montrent qu'après le traitement par un bisphosphonate :

- le volume trabeculaire osseux n'a pas été significativement diminué par le traitement,
- les qualités biomécaniques sont améliorées, en effet : la rigidité osseuse et la résistance à la rupture sont augmentées, surtout avec le tiludronate.

EXEMPLE 2 : Comprimé pour l'amélioration de la réparation osseuse.

| Comprimé sécable : | |
|---|---|
| - sel disodique d'acide tiludronique correspondant à 200 mg d'acide | 240 mg |
| - laurylsulfate de sodium | 4,5 mg |
| - carboxyméthylcellulose sodique réticulée | 24 mg |
| - lactose microcristallin | 177 mg |
| - stéarate de magnésium | 4,5 mg |
| | 450 mg |

EXEMPLE 3 : Comprimé pour l'amélioration de la réparation osseuse.

| Comprimé sécable : | |
|---|---|
| - sel disodique d'acide tiludronique correspondant à 50 mg d'acide | 60 mg |
| - laurylsulfate de sodium | 3 mg |
| - carboxyméthylcellulose sodique réticulée | 6 mg |
| - lactose anhydre | 44,25 mg |
| - stéarate de magnésium | 1,25 mg |
| | 114,5 mg |

EXEMPLE 4 : Comprimé pour l'amélioration de la réparation osseuse

| | |
|---|---|
| - sel disodique de l'acide tiludronique correspondant à 25 mg d'acide | 30 mg |
| - carboxyméthylcellulose sodique réticulée | 3 mg |
| - stéarate de magnésium | 0,75 mg |
| - lactose microcristallin | 22,25 mg |
| | 56 mg |

EXEMPLE 5 : Solution injectable pour amélioration de la réparation osseuse

| | |
|---|---|
| - sel disodique de l'acide étidronique | 300 mg |
| - eau pour injection | |
| | 6 ml |

EXEMPLE 6 : Préparation pour perfusion pour amélioration de la réparation osseuse

| | |
|---|---|
| - lyophilisat de pamidronate de sodium : | 15 mg |
| - eau pour injection | 6 ml |

EXEMPLE 7 : Préparation pour perfusion pour amélioration de la réparation osseuse

| | |
|---|---|
| - sel disodique de l'acide clodronique | 300 mg |
| - hydroxyde de sodium | q.s.p. pH 5 |
| - eau pour injection | |
| | 5 ml |

## Revendications

### Revendications pour les Etats contractants suivants : AT, BE, CH, DK, ES, GR, IE, IT, LI, LU, NL, PT, SE

**1.** Utilisation d'un dérivé de l'acide bisphosphonique de formule :

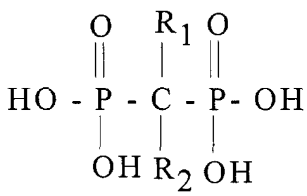

(I)

dans laquelle :

- $R_1$ représente un atome d'hydrogène, un atome d'halogène, un hydroxyle, un amino, un monoalkyl($C_1$-$C_4$) amino, un dialkyl($C_1$-$C_4$)amino ;
- $R_2$ représente un atome d'halogène, un alkyle linéaire comportant de 1 à 5 atomes de carbone non substitué ou substitué par un groupe choisi parmi un atome de chlore, un hydroxyle, un amino, un monoalkyl($C_1$-$C_4$) amino, un dialkyl($C_1$-$C_4$)amino, un cycloalkyl($C_3$-$C_7$) amino, ou $R_2$ représente un phénoxy, un phényle, un thiol, un phénylthio, un chlorophénylthio, un pyridyle, un pyridylméthyle, un 1-pyridyl-1-hydroxyméthyle, un imidazolylméthyle, un thiomorpholin-4-yle ;

et de ses sels avec des acides minéraux ou organiques pharmaceutiquement acceptables pour la préparation de médicaments destinés à favoriser la réparation osseuse; lesdits médicaments étant destinés à une administration orale et contenant également du laurylsulfate de sodium.

**2.** Utilisation selon la revendication 1 dans laquelle le médicament contient de 0,001 mg à 400 mg de dérivé(s) d'acide bisphosphonique de formule (I).

**3.** Utilisation selon l'une des revendications 1 ou 2 dans laquelle le médicament contient de 0,1 à 250 mg de dérivé (s) d'acide bisphophonique de formule (I).

**4.** Utilisation selon l'une quelconque des revendications 1 à 3 de :

- l'acide 2-pyridin-2-yl éthylidène bisphosphonique dont la dénomination commune internationale est l'acide piridronique et ses sels de sodium ;
- l'acide dichlorométhylène bisphosphonique dont la dénomination commune internationale est l'acide clodronique et ses sels de sodium ;
- l'acide 3-amino-1-hydroxy propylidène bisphosphonique dont la dénomination commune internationale est l'acide pamidronique et ses sels de sodium ;
- l'acide 4-amino-1-hydroxy butylidène bisphosphonique dont la dénomination commune internationale est acide alendronique et ses sels de sodium ;
- l'acide 6-amino-1-hydroxy hexylidène bisphosphonique et ses sels pharmaceutiquement acceptables ;
- l'acide phénoxy méthylène bisphosphonique et ses sels pharmaceutiquement acceptables;

- l'acide thiomorpholino méthylène bisphosphonique et ses sels pharmaceutiquement acceptables ;
- l'acide 4-chloro phénylthiométhylène bisphosphonique dont la dénomination commune internationale est acide tiludronique et ses sels pharmaceutiquement acceptables ; notamment le sel disodique ;
- l'acide 1-hydroxy-2-(pyridin-3-yl)éthylidène bisphosphonique dont la dénomination commune internationale est acide risédronique et ses sels de sodium ;
- l'acide 1-hydroxy-2-(imidazol-2-yl)éthyl-1,1-bisphosphonique et ses sels pharmaceutiquement acceptables ;
- l'acide (cycloheptylamino)méthylène bisphosphonique et ses sels pharmaceutiquement acceptables ;
- l'acide 2-hydroxyéthylidène-2-(pyridin-3-yl)-1,1-bisphosphonique et ses sels de sodium.

**5.** Utilisation d'un dérivé de l'acide bisphophonique, choisi parmi l'acide étidronique et ses sels pharmaceutiquement acceptables et le sel disodique de l'acide tiludronique, pour la préparation de médicaments destinés à favoriser la réparation osseuse.

**6.** Utilisation selon la revendication 5 dans laquelle le médicament contient de 0,001 mg à 400 mg de dérivé(s) d'acide bisphophonique selon ladite revendication 5.

**7.** Utilisation selon l'une des revendications 5 ou 6 dans laquelle le médicament contient de 0,1 à 250 mg de dérivé (s) d'acide bisphophonique selon ladite revendication 5.

**8.** Utilisation selon l'une quelconque des revendications 5 à 7 pour la préparation de médicaments destinés à une administration percutanée.

**9.** Utilisation selon l'une quelconque des revendications 5 à 7 pour la préparation de médicaments destinés à une administration orale.

**10.** Utilisation selon la revendication 9, pour la préparation de comprimés ayant la composition suivante, pour 450 mg :

| | |
|---|---|
| - sel disodique d'acide tiludroniquecorrespondant à 200 mg d'acide | 240 mg |
| - laurylsulfate de sodium | 4,5 mg |
| - carboxyméthylcellulose sodique réticulée | 24 mg |
| - lactose microcristallin | 177 mg |
| - stéarate de magnésium | 4,5 mg |

**11.** Utilisation selon la revendication 9 pour la préparation de comprimés ayant la composition suivante, pour 114,5 mg :

| | |
|---|---|
| - sel disodique d'acide tiludronique correspondant à 50 mg d'acide | 60 mg |
| - laurylsulfate de sodium | 3 mg |
| - carboxyméthylcellulose sodique réticulée | 6 mg |
| - lactose anhydre | 44,25 mg |
| - stéarate de magnésium | 1,25 mg |

**Revendications pour les Etats contractants suivants : DE, FR, GB**

**1.** Utilisation d'un dérivé de l'acide bisphosphonique de formule :

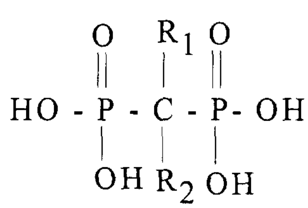

(I)

dans laquelle :

- $R_1$ représente un atome d'hydrogène, un atome d'halogène, un hydroxyle, un amino, un monoalkyl($C_1$-$C_4$) amino, un dialkyl($C_1$-$C_4$)amino ;
- $R_2$ représente un atome d'halogène, un alkyle linéaire comportant de 1 à 5 atomes de carbone non substitué ou substitué par un groupe choisi parmi un atome de chlore, un hydroxyle, un amino, un monoalkyl($C_1$-$C_4$) amino, un dialkyl($C_1$-$C_4$)amino, un cycloalkyl($C_3$-$C_7$) amino, ou $R_2$ représente un phénoxy, un phényle, un thiol, un phénylthio, un chlorophénylthio, un pyridyle, un pyridylméthyle, un 1-pyridyl-1-hydroxyméthyle, un imidazolylméthyle, un thiomorpholin-4-yle ;

et de ses sels avec des acides minéraux ou organiques pharmaceutiquement acceptables pour la préparation de médicaments destinés à favoriser la réparation osseuse; lesdits médicaments étant destinés à une administration orale et contenant également du laurylsulfate de sodium.

**2.** Utilisation selon la revendication 1 dans laquelle le médicament contient de 0,001 mg à 400 mg de dérivé(s) d'acide bisphosphonique de formule (I).

**3.** Utilisation selon l'une des revendications 1 ou 2 dans laquelle le médicament contient de 0,1 à 250 mg de dérivé (s) d'acide bisphophonique de formule (I).

**4.** Utilisation selon l'une quelconque des revendications 1 à 3 de :

- l'acide 2-pyridin-2-yl éthylidène bisphosphonique dont la dénomination commune internationale est l'acide piridronique et ses sels de sodium ;
- l'acide dichlorométhylène bisphosphonique dont la dénomination commune internationale est l'acide clodronique et ses sels de sodium ;
- l'acide 3-amino-1-hydroxy propylidène bisphosphonique dont la dénomination commune internationale est l'acide pamidronique et ses sels de sodium ;
- l'acide 4-amino-1-hydroxy butylidène bisphosphonique dont la dénomination commune internationale est acide alendronique et ses sels de sodium ;
- l'acide 6-amino-1-hydroxy hexylidène bisphosphonique et ses sels pharmaceutiquement acceptables ;
- l'acide phénoxy méthylène bisphosphonique et ses sels pharmaceutiquement acceptables;
- l'acide thiomorpholino méthylène bisphosphonique et ses sels pharmaceutiquement acceptables ;
- l'acide 4-chloro phénylthiométhylène bisphosphonique dont la dénomination commune internationale est acide tiludronique et ses sels pharmaceutiquement acceptables ; notamment le sel disodique ;
- l'acide 1-hydroxy-2-(pyridin-3-yl)éthylidène bisphosphonique dont la dénomination commune internationale est acide risédronique et ses sels de sodium ;
- l'acide 1-hydroxy-2-(imidazol-2-yl)éthyl-1,1-bisphosphonique et ses sels pharmaceutiquement acceptables ;
- l'acide (cycloheptylamino)méthylène bisphosphonique et ses sels pharmaceutiquement acceptables;
- l'acide 2-hydroxyéthylidène-2-(pyridin-3-yl)-1,1-bisphosphonique et ses sels de sodium.

**5.** Utilisation selon l'une quelconque des revendications 1 à 4, pour la préparation de comprimés ayant la composition suivante, pour 450 mg :

| | |
|---|---|
| - sel disodique d'acide tiludronique correspondant à 200 mg d'acide | 240 mg |
| - laurylsulfate de sodium | 4,5 mg |
| - carboxyméthylcellulose sodique réticulée | 24 mg |
| - lactose microcristallin | 177 mg |
| - stéarate de magnésium | 4,5 mg |

**6.** Utilisation selon l'une quelconque des revendications 1 à 4, pour la préparation de comprimés ayant la composition suivante, pour 114,5 mg :

| | |
|---|---|
| - sel disodique d'acide tiludronique correspondant à 50 mg d'acide | 60 mg |
| - laurylsulfate de sodium | 3 mg |
| - carboxyméthylcellulose sodique réticulée | 6 mg |
| - lactose anhydre | 44,25 mg |
| - stéarate de magnésium | 1,25 mg |

## Claims

**Claims for the following Contracting States : AT, BE, CH, DK, ES, GR, IE, IT, LI LU, NL, PT, SE**

1. Use of a bisphosphonic acid derivative of the formula

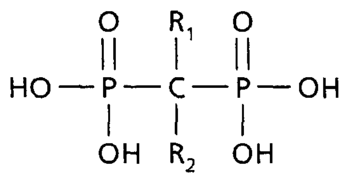

(I)

in which:

- $R_1$ is a hydrogen atom, a halogen atom, a hydroxyl, an amino, a mono-$C_1$-$C_4$-alkylamino, a di-$C_1$-$C_4$-alkylamino; and
- $R_2$ is a halogen atom, a linear alkyl containing from 1 to 5 carbon atoms which is unsubstituted or substituted by a group selected from a chlorine atom, a hydroxyl, an amino, a mono-$C_1$-$C_4$-alkylamino, a di-$C_1$-$C_4$-alkylamino, a $C_3$-$C_7$-cycloalkylamino, or $R_2$ is a phenoxy, a phenyl, a thiol, a phenylthio, a chlorophenylthio, a pyridyl, a pyridylmethyl, a 1-pyridyl-1-hydroxymethyl, an imidazolylmethyl, a thiomorpholin-4-yl ;

and of its salts with pharmaceutically acceptable mineral or organic acids for the preparation of drugs designed to promote bone repair; said drugs being intended for oral administration and also containing sodium laurylsulfate.

2. Use according to claim 1 in which the drug contains from 0.001 mg to 400 mg of bisphosphonic acid derivative(s) of formula (I).

3. Use according to claim 1 or 2 in which the drug contains from 0.1 to 250 mg of bisphosphonic acid derivative(s) of formula (I).

4. Use according to any one of claims 1 to 3 of:

- 2-pyridin-2-ylethylidenebisphosphonic acid, having the international non-proprietary name piridronic acid, and its sodium salts ;
- dichloromethylenebisphosphonic acid, having the international non-proprietary name clodronic acid, and its sodium salts;
- 3-amino-1-hydroxypropylidenebisphosphonic acid, having the international non-proprietary name pamidronic acid, and its sodium salts ;
- 4-amino-1-hydroxybutylidenebisphosphonic acid, having the international non-proprietary name alendronic acid, and its sodium salts ;
- 6-amino-1-hydroxyhexylidenebisphosphonic acid and its pharmaceutically acceptable salts;
- phenoxymethylenebisphosphonic acid and its pharmaceutically acceptable salts;
- thiomorpholinomethylenebisphosphonic acid and its pharmaceutically acceptable salts;
- 4-chlorophenylthiomethylenebisphosphonic acid, having the non-proprietary name tiludronic acid, and its pharmaceutically acceptable salts, especially the disodium salt;
- 1-hydroxy-2-(pyridin-3-yl)ethylidenebisphosphonic acid, having the international non-proprietary name risedronic acid, and its sodium salts;
- 1-hydroxy-2-(imidazol-2-yl)ethyl-1,1-bisphosphonic acid and its pharmaceutically acceptable salts;
- (cycloheptylamino)methylenebisphosphonic acid and its pharmaceutically acceptable salts; and
- 2-hydroxyethylidene-2-(pyridin-3-yl)-1,1-bisphosphonic acid and its sodium salts.

5. Use of a bisphosphonic acid derivative, selected from etidronic acid and its pharmaceutically acceptable salts and the disodium salt of the tiludronic acid, for the preparation of drugs designed to promote bone repair.

6. Use according to claim 5 in which the drug contains from 0.001 mg to 400 mg of bisphosphonic acid derivative(s)

according to said claim 5.

**7.** Use according to claim 5 or 6 in which the drug contains from 0.1 to 250 mg of bisphosphonic acid derivative(s) according to said claim 5.

**8.** Use according to any one of claims 5 to 7 for the preparation of drugs intended for transdermal administration.

**9.** Use according to any one of claims 5 to 7 for the preparation of drugs intended for oral administration.

**10.** Use according to claim 9, for the preparation of tablets having the following composition, for 450 mg:

| | |
|---|---|
| - disodium salt of tiludronic acid corresponding to 200 mg of acid | 240 mg |
| - sodium laurylsulfate | 4.5 mg |
| - crosslinked sodium carboxymethyl cellulose | 24 mg |
| - microcrystalline lactose | 177 mg |
| - magnesium stearate | 4.5 mg |

**11.** Use according to claim 9, for the preparation of tablets having the following composition, for 114.5 mg:

| | |
|---|---|
| - disodium salt of tiludronic acid corresponding to 50 mg of acid | 60 mg |
| - sodium laurylsulfate | 3 mg |
| - crosslinked sodium carboxymethyl cellulose | 6 mg |
| - anhydrous lactose | 44.25 mg |
| - magnesium stearate | 1.25 mg |

**Claims for the following Contracting States : DE, FR, GB**

**1.** Use of a bisphosphonic acid derivative of the formula

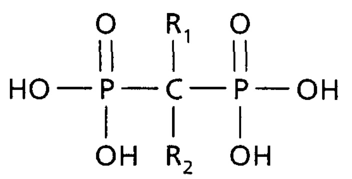

(I)

in which:

- $R_1$ is a hydrogen atom, a halogen atom, a hydroxyl, an amino, a mono-$C_1$-$C_4$-alkylamino, a di-$C_1$-$C_4$-alkylamino; and
- $R_2$ is a halogen atom, a linear alkyl containing from 1 to 5 carbon atoms which is unsubstituted or substituted by a group selected from a chlorine atom, a hydroxyl, an amino, a mono-$C_1$-$C_4$-alkylamino, a di-$C_1$-$C_4$-alkylamino, a $C_3$-$C_7$-cycloalkylamino, or $R_2$ is a phenoxy, a phenyl, a thiol, a phenylthio, a chlorophenylthio, a pyridyl, a pyridylmethyl, a 1-pyridyl-1-hydroxymethyl, an imidazolylmethyl, a thiomorpholin-4-yl;

and of its salts with pharmaceutically acceptable mineral or organic acids for the preparation of drugs designed to promote bone repair; said drugs being intended for oral administration and also containing sodium laurylsulfate.

**2.** Use according to claim 1 in which the drug contains from 0.001 mg to 400 mg of bisphosphonic acid derivative(s) of formula (I).

**3.** Use according to claim 1 or 2 in which the drug contains from 0.1 to 250 mg of bisphosphonic acid derivative(s) of formula (I).

**4.** Use according to any one of claims 1 to 3 of:

- 2-pyridin-2-ylethylidenebisphosphonic acid, having the international non-proprietary name piridronic acid, and its sodium salts ;
- dichloromethylenebisphosphonic acid, having the international non-proprietary name clodronic acid, and its sodium salts ;
- 3-amino-1-hydroxypropylidenebisphosphonic acid, having the international non-proprietary name pamidronic acid, and its sodium salts;
- 4-amino-1-hydroxybutylidenebisphosphonic acid, having the international non-proprietary name alendronic acid, and its sodium salts;
- 6-amino-1-hydroxyhexylidenebisphosphonic acid and its pharmaceutically acceptable salts;
- phenoxymethylenebisphosphonic acid and its pharmaceutically acceptable salts;
- thiomorpholinomethylenebisphosphonic acid and its pharmaceutically acceptable salts;
- 4-chlorophenylthiomethylenebisphosphonic acid, having the non-proprietary name tiludronic acid, and its pharmaceutically acceptable salts, especially the disodium salt;
- 1-hydroxy-2-(pyridin-3-yl)ethylidenebisphosphonic acid, having the international non-proprietary name risedronic acid, and its sodium salts ;
- 1-hydroxy-2-(imidazol-2-yl)ethyl-1,1-bisphosphonic acid and its pharmaceutically acceptable salts ;
- (cycloheptylamino)methylenebisphosphonic acid and its pharmaceutically acceptable salts; and
- 2-hydroxyethylidene-2-(pyridin-3-yl)-1,1-bisphosphonic acid and its sodium salts.

**5.** Use according to any one of claims 1 to 4, for the preparation of tablets having the following composition, for 450 mg:

| | |
|---|---|
| - disodium salt of tiludronic acid corresponding to 200 mg of acid | 240 mg |
| - sodium laurylsulfate | 4.5 mg |
| - crosslinked sodium carboxymethyl cellulose | 24 mg |
| - microcrystalline lactose | 177 mg |
| - magnesium stearate | 4.5 mg |

**6.** Use according to any one of claims 1 to 4, for the preparation of tablets having the following composition, for 114.5 mg:

| | |
|---|---|
| - disodium salt of tiludronic acid corresponding to 50 mg of acid | 60 mg |
| - sodium laurylsulfate | 3 mg |
| - crosslinked sodium carboxymethyl cellulose | 6 mg |
| - anhydrous lactose | 44.25 mg |
| - magnesium stearate | 1.25 mg |

**Patentansprüche**

**Patentansprüche für folgende Vertragsstaaten : AT, BE, CH, DK, ES, GR, IE, IT, LI, LU, NL, PT, SE**

**1.** Verwendung eines Bisphosphonsäure-Derivats der Formel:

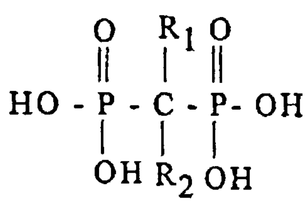

(I)

worin:

- $R_1$ ein Wasserstoffatom, ein Halogenatom, Hydroxyl, Amino, Monoalkyl-($C_1$-$C_4$)-amino, Dialkyl-($C_1$-$C_4$)-amino darstellt;
- $R_2$ ein Wasserstoffatom, ein lineares Alkyl mit 1 bis 5 Kohlenstoffatomen, das gegebenenfalls durch eine Gruppe ausgewählt aus einem Chloratom, Hydroxyl, Amino, Monoalkyl-($C_1$-$C_4$)-amino, Dialkyl-($C_1$-$C_4$)-amino, Cycloalkyl-($C_3$-$C_7$)-amino substituiert ist, oder $R_2$ Phenoxy, Phenyl, Thiol, Phenylthio, Chlorphenylthio, Pyridyl, Pyridylmethyl, 1-Pyridyl-1-hydroxymethyl, Imidazolylmethyl, Thiomorpholin-4-yl darstellt;

und seiner Salze mit pharmazeutisch annehmbaren Mineral- oder organischen Säuren zur Herstellung von Medikamenten zur Förderung des Wiederaufbaus von Knochen, welche Medikamente für orale Verabreichung bestimmt sind und auch Natriumlaurylsulfat enthalten.

**2.** Verwendung nach Anspruch 1, worin das Medikament 0,001 mg bis 400 mg Bisphosphonsäure-Derivat(e) der Formel (I) enthält.

**3.** Verwendung nach einem der Ansprüche 1 oder 2, worin das Medikament 0,1 bis 250 mg Bisphosphonsäure-Derivat(e) der Formel (I) enthält.

**4.** Verwendung nach einem der Ansprüche 1 bis 3, von:

- 2-Pyridin-2-yl-ethylidenbisphosphonsäure, deren allgemeine internationale Bezeichnung Piridronsäure ist, und ihren Natriumsalzen;
- Dichlormethylenbisphosphonsäure, deren allgemeine internationale Bezeichnung Clodronsäure ist, und ihren Natriumsalzen;
- 3-Amino-1-hydroxypropylidenbisphosphonsäure, deren allgemeine internationale Bezeichnung Pamidronsäure ist, und ihren Natriumsalzen;
- 4-Amino-1-hydroxybutylidenbisphosphonsäure, deren allgemeine internationale Bezeichnung Alendronsäure ist, und ihren Natriumsalzen;
- 6-Amino-1-hydroxyhexylidenbisphosphonsäure und ihren pharmazeutisch annehmbaren Salzen;
- Phenoxymethylenbisphosphonsäure und ihren pharmazeutisch annehmbaren Salzen;
- Thiomorpholinomethylenbisphosphonsäure und ihren pharmazeutisch annehmbaren Salzen;
- 4-Chlorphenylthiomethylenbisphosphonsäure, deren allgemeine internationale Bezeichnung Tiludronsäure ist, und ihren pharmazeutisch annehmbaren Salzen, insbesondere dem Dinatriumsalz;
- 1-Hydroxy-2-(pyridin-3-yl)-ethylidenbisphosphonsäure, deren allgemeine internationale Bezeichnung Risedronsäure ist, und ihren Natriumsalzen;
- 1-Hydroxy-2-(imidazol-2-yl)-ethyl-1,1-bisphosphonsäure und ihren pharmazeutisch annehmbaren Salzen;
- (Cycloheptylamino)-methylenbisphosphonsäure und ihren pharmazeutisch annehmbaren Salzen;
- 2-Hydroxyethyliden-2-(pyridin-3-yl)-1,1-bisphosphonsäure und ihren Natriumsalzen.

**5.** Verwendung eines Bisphosphonsäure-Derivats ausgewählt aus Etidronsäure und ihren pharmazeutisch annehmbaren Salzen und dem Dinatriumsalz von Tiludronsäure zur Herstellung von Medikamenten zur Förderung des Wiederaufbaus von Knochen.

**6.** Verwendung nach Anspruch 5, worin das Medikament 0,001 mg bis 400 mg Bisphosphonsäure-Derivat(e) nach Anspruch 5 enthält.

**7.** Verwendung nach einem der Ansprüche 5 oder 6, worin das Medikament 0,1 bis 250 mg Bisphosphonsäure-Derivat(e) nach Anspruch 5 enthält.

**8.** Verwendung nach einem der Ansprüche 5 bis 7 zur Herstellung von für perkutane Verabreichung bestimmten Medikamenten.

**9.** Verwendung nach einem der Ansprüche 5 bis 7 zur Herstellung von für orale Verabreichung bestimmten Medikamenten.

**10.** Verwendung nach Anspruch 9 zur Herstellung von Tabletten mit folgender Zusammensetzung pro 450 mg:

| | |
|---|---|
| - Tiludronsäuredinatriumsalz entsprechend 200 mg Säure | 240 mg |

(fortgesetzt)

| | |
|---|---|
| - Natriumlaurylsulfat | 4,5 mg |
| - vernetzte Natriumcarboxymethylcellulose | 24 mg |
| - mikrokristalline Lactose | 177 mg |
| - Magnesiumstearat | 4,5 mg |

**11.** Verwendung nach Anspruch 9 zur Herstellung von Tabletten mit folgender Zusammensetzung pro 114,5 mg:

| | |
|---|---|
| - Tiludronsäuredinatriumsalz entsprechend 50 mg Säure | 60 mg |
| - Natriumlaurylsulfat | 3 mg |
| - vernetzte Natriumcarboxymethylcellulose | 6 mg |
| - wasserfreie Lactose | 44,25 mg |
| - Magnesiumstearat | 1,25 mg |

**Patentansprüche für folgende Vertragsstaaten : DE, FR, GB**

**1.** Verwendung eines Bisphosphonsäure-Derivats der Formel:

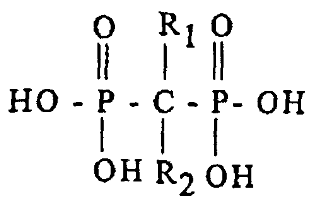

(I)

worin:

- $R_1$ ein Wasserstoffatom, ein Halogenatom, Hydroxyl, Amino, Monoalkyl-($C_1$-$C_4$)-amino, Dialkyl-($C_1$-$C_4$)-amino darstellt;
- $R_2$ ein Wasserstoffatom, ein lineares Alkyl mit 1 bis 5 Kohlenstoffatomen, das gegebenenfalls durch eine Gruppe ausgewählt aus einem Chloratom, Hydroxyl, Amino, Monoalkyl-($C_1$-$C_4$)-amino, Dialkyl-($C_1$-$C_4$)-amino, Cycloalkyl-($C_3$-$C_7$)-amino substituiert ist, oder $R_2$ Phenoxy, Phenyl, Thiol, Phenylthio, Chlorphenylthio, Pyridyl, Pyridylmethyl, 1-Pyridyl-1-hydroxymethyl, Imidazolylmethyl, Thiomorpholin-4-yl darstellt; und seiner Salze mit pharmazeutisch annehmbaren Mineral- oder organischen Säuren zur Herstellung von Medikamenten zur Förderung des Wiederaufbaus von Knochen, welche Medikamente für orale Verabreichung bestimmt sind und auch Natriumlaurylsulfat enthalten.

**2.** Verwendung nach Anspruch 1, worin das Medikament 0,001 mg bis 400 mg Bisphosphonsäure-Derivat(e) der Formel (I) enthält.

**3.** Verwendung nach einem der Ansprüche 1 oder 2, worin das Medikament 0,1 bis 250 mg Bisphosphonsäure-Derivat(e) der Formel (I) enthält.

**4.** Verwendung nach einem der Ansprüche 1 bis 3, von:

- 2-Pyridin-2-yl-ethylidenbisphosphonsäure, deren allgemeine internationale Bezeichnung Piridronsäure ist, und ihren Natriumsalzen;
- Dichlormethylenbisphosphonsäure, deren allgemeine internationale Bezeichnung Clodronsäure ist, und ihren Natriumsalzen;
- 3-Amino-1-hydroxypropylidenbisphosphonsäure, deren allgemeine internationale Bezeichnung Pamidronsäure ist, und ihren Natriumsalzen;
- 4-Amino-1-hydroxybutylidenbisphosphonsäure, deren allgemeine internationale Bezeichnung Alendronsäure ist, und ihren Natriumsalzen;

- 6-Amino-1-hydroxyhexylidenbisphosphonsäure und ihren pharmazeutisch annehmbaren Salzen;
- Phenoxymethylenbisphosphonsäure und ihren pharmazeutisch annehmbaren Salzen;
- Thiomorpholinomethylenbisphosphonsäure und ihren pharmazeutisch annehmbaren Salzen;
- 4-Chlorphenylthiomethylenbisphosphonsäure, deren allgemeine internationale Bezeichnung Tiludronsäure ist, und ihren pharmazeutisch annehmbaren Salzen, insbesondere dem Dinatriumsalz;
- 1-Hydroxy-2-(pyridin-3-yl)-ethylidenbisphosphonsäure, deren allgemeine internationale Bezeichnung Risedronsäure ist, und ihren Natriumsalzen;
- 1-Hydroxy-2-(imidazol-2-yl)-ethyl-1,1-bisphosphonsäure und ihren pharmazeutisch annehmbaren Salzen;
- (Cycloheptylamino)-methylenbisphosphonsäure und ihren pharmazeutisch annehmbaren Salzen;
- 2-Hydroxyethyliden-2-(pyridin-3-yl)-1,1-bisphosphonsäure und ihren Natriumsalzen.

**5.** Verwendung nach einem der Ansprüche 1 bis 4 zur Herstellung von Tabletten mit folgender Zusammensetzung pro 450 mg:

| | |
|---|---|
| - Tiludronsäuredinatriumsalz entsprechend 200 mg Säure | 240 mg |
| - Natriumlaurylsulfat | 4,5 mg |
| - vernetzte Natriumcarboxymethylcellulose | 24 mg |
| - mikrokristalline Lactose | 177 mg |
| - Magnesiumstearat | 4,5 mg |

**6.** Verwendung nach einem der Ansprüche 1 bis 4 zur Herstellung von Tabletten mit folgender Zusammensetzung pro 114,5 mg:

| | |
|---|---|
| - Tiludronsäuredinatriumsalz entsprechend 50 mg Säure | 60 mg |
| - Natriumlaurylsulfat | 3 mg |
| - vernetzte Natriumcarboxymethylcellulose | 6 mg |
| - wasserfreie Lactose | 44,25 mg |
| - Magnesiumstearat | 1,25 mg |